# FASCICULE DE BREVET EUROPEEN

(11) **EP 1 796 738 B1**
(45) Date de publication et mention de la délivrance du brevet: **07.08.2013**
(21) Numéro de dépôt: 05786927.3
(22) Date de dépôt: 23.09.2005
(51) Int. Cl.: A61K 49/10, A61K 51/04, A61K 103/00

(54) **CONJUGUÉ DE BICYCLAM POUR L'IRM ET LA SCINTIGRAPHIE DE MALADIES ASSOCIÉES AU RÉCEPTEUR CXCR4**
BICYCLAM KONJUGAT FÜR DIE MRT UND SZINTIGRAPHIE VON KRANKHEITEN ASSOZIERT MIT DEM CXCR4 REZEPTOR
BICYCLAM CONJUGATE FOR MRI AND SCINTIGRAPHY OF DISEASES ASSOCIATED WITH THE CXCR4 RECEPTOR

(30) Priorité: 23.09.2004 FR 0410062
(43) Date de publication de la demande: 20.06.2007
(73) Titulaire: GUERBET, 93420 Villepinte (FR)
(72) Inventeur: PORT, Marc, F-95170 Deuil La Barre (FR)
(74) Mandataire: Regimbeau
(86) Numéro de dépôt international: PCT/EP2005/054797
(87) Numéro de publication internationale: WO 2006/032704

(56) Documents cités:
- WO-A-2004/054615
- US-A1- 2004 102 428
- MUKAI T ET AL: "Synthesis and evaluation of indium-111-labeled peptide derivative targeted to a chemokine receptor, CXCR4." JOURNAL OF LABELLED COMPOUNDS AND RADIOPHARMACEUTICALS, vol. 46, no. Supplement 1, août 2003 (2003-08), page S304, XP002398876 & 15TH INTERNATIONAL SYMPOSIUM ON RADIOPHARMACEUTICAL CHEMISTRY; SYDNEY, AUSTRALIA; AUGUST 10-14, 2003 ISSN: 0362-4803
- LIANG ZHONGXING ET AL: "Inhibition of breast cancer metastasis by selective synthetic polypeptide against CXCR4." CANCER RESEARCH. 15 JUN 2004, vol. 64, no. 12, 15 juin 2004 (2004-06-15), pages 4302-4308, XP002326726 ISSN: 0008-5472 cité dans la demande
- TAMAMURA H ET AL: "A low-molecular-weight inhibitor against the chemokine receptor CXCR4: A strong anti-HIV peptide T140" BIOCHEMICAL AND BIOPHYSICAL RESEARCH COMMUNICATIONS, ACADEMIC PRESS INC. ORLANDO, FL, US, vol. 253, 1998, pages 877-882, XP002169961 ISSN: 0006-291X
- LARS OLE GERLACH: "Molecular interactions of cyclam and bicyclam non-peptide antagonists with the CXCR4 chemokine receptor" JOURNAL OF BIOLOGICAL CHEMISTRY, AMERICAN SOCIETY OF BIOLOCHEMICAL BIOLOGISTS, BIRMINGHAM,, US, vol. 276, no. 17, 27 avril 2001 (2001-04-27), pages 14153-14160, XP002357019 ISSN: 0021-9258
- DESSOLIN ET AL: "New Bicyclam-AZT Conjugates: Design, Synthesis, Anti-HIV Evaluation, and Their Interaction with CXCR-4 Coreceptor" JOURNAL OF MEDICINAL CHEMISTRY, AMERICAN CHEMICAL SOCIETY. WASHINGTON, US, vol. 42, no. 2, 1999, pages 229-241, XP002199037 ISSN: 0022-2623 cité dans la demande
- LE BON B ET AL: "AMD3100 Conjugates as Components of Targeted Nonviral Gene Delivery Systems: Synthesis and in Vitro Transfection Efficiency of CXCR4-Expressing Cells" BIOCONJUGATE CHEMISTRY 2004 UNITED STATES, vol. 15, no. 2, 2004, pages 413-423, XP002398953 ISSN: 1043-1802 cité dans la demande
- ICHIYAMA K ET AL: "A duodenally absorbable CXC chemokine receptor 4 antagonist, KRH-1636, exhibits a potent and selective anti-HIV-1 activity" PROCEEDINGS OF THE NATIONAL ACADEMY OF SCIENCES OF THE UNITED STATES OF AMERICA 01 APR 2003 UNITED STATES, vol. 100, no. 7, 1 avril 2003 (2003-04-01), pages 4185-4190, XP002398954 ISSN: 0027-8424 cité dans la demande

## Description

L'invention concerne des composés de diagnostique destinés à cibler des récepteurs aux chimiokines notamment les récepteurs CXCR4, et l'utilisation de ces composés dans le domaine de l'imagerie médicale.

Les chimiokines sont des molécules de la famille des cytokines, qui présentent des propriétés d'activation, en particulier de cellules de la famille des leucocytes faisant intervenir notamment des propriétés chemoattractives, des propriétés de mobilisation du calcium par augmentation du calcium intracellulaire et des propriétés de relargage d'enzymes (exocytose). Ces chimiokines sont connues pour leur rôle éventuel en tant que médiateurs de l'inflammation. Plusieurs chimiokines ont été décrites par référence à leur structure et à leur affinité pour un ou plusieurs récepteurs et par référence à leurs propriétés biologiques, dans une publication de Baggiolini M. et al (Advances in Immunology (1994), vol. 55, pages 97-179). Une publication de Wells T.N.C. et al (Journal of Leukocyte Biology, vol. 59, January 1996, 53-60) décrit la structure tridimensionnelle de plusieurs chimiokines et leurs récepteurs spécifiques ou non. De façon générale, ces chimiokines sont caractérisées par la présence dans leur structure primaire, de résidus cystéine conservés (1 à 4 résidus notamment) sur la base desquels plusieurs sous-familles ont été distinguées selon la position des deux premières cystéines. Ces familles comprennent celles des protéines CXC ou des protéines CC. La présence de ces résidus cystéine induit la formation de ponts disulfure.

Le rôle déterminant des chimiokines et de leurs récepteurs a été démontré dans la prolifération cancéreuse due aux métastases, notamment des carcinomes pulmonaires, (Muller et al, Nature 410: 50-6 (2001), WO 99/47518). Des cellules malignes issues de tumeurs primaires vont coloniser d'autres tissus en passant par la circulation sanguine ou lymphatique, des chimiokines intervenant dans ce processus. Les mécanismes moléculaires impliqués dans la progression et la métastase tumorale (la mobilité, la migration, la prolifération cellulaire, l'adhésion de celles cancéreuses circulantes aux cellules endothéliales) ne sont pas totalement élucidés. Le rôle des récepteurs CXCR4 est toutefois démontré, et des ligands capables de cibler des récepteurs CXCR4 et ainsi d'inhiber la progression tumorale ont été décrits, notamment dans le document US 2004/0132642.

L'invention concerne le ciblage de récepteurs CXCR4, non pour le traitement, mais pour le diagnostique d'une pathologie cancéreuse, et en particulier l'évaluation des risques et du stade de la progression des métastases.

Mukai et al (J. Label Compd. Radiopharm. 2003: 46: S1-S403, page S304) ont décrit l'utilisation du composé ¹¹¹In-DTPA-Ac-TZ14011 basé sur le peptide TZ14011 ciblant CXCR4 pour le radiodiagnostic de tumeurs métastatiques.

L'homme du métier connaît pour l'IRM notamment (Imagerie par Résonance Magnétique) un grand nombre de produits de contraste, dits non spécifiques, à base de chélates de métal paramagnétique tel que le Gadolinium, linéaires ou macrocycliques, notamment DTPA, DTPA BMA, DTPA BOPTA, DO3A, TETA, TRITA, HETA, DOTA-NHS, TETA-NHS, PCTA, DOTA, M4DOTA, M4DO3A, M4DOTMA, MPDO3A, HBED, EHPG, BFCs. Mais ces composés ne permettent toutefois pas de reconnaître spécifiquement une zone pathologique, et en particulier les métastases tumorales.

Par opposition à un produit non spécifique de l'art antérieur (sans ciblage de marqueur biologique), les composés selon l'invention visent à identifier spécifiquement des cibles biologiques (cellules, tissus) présentant une surexpression des récepteurs CXCR4 par rapport à une zone non pathologique. En particulier, les composés selon l'invention sont destinés à cibler des cellules tumorales exprimant les récepteurs CXCR4, et de manière préférée des cellules métastatiques.

Ainsi selon un premier aspect, l'invention concerne des composés comprenant d'une part une partie biovecteur de ciblage des CXCR4, et d'autre part une partie de détection (entité signal) capable d'être identifié par une méthode d'imagerie médicale.

La partie de détection est typiquement un agent de contraste détectable par imagerie IRM, rayons X, scintigraphie aux rayons gamma, scanner CT, PET, imagerie CEST et notamment Lipocest (nanoparticules lipidiques exposées à une imagerie CEST).

Dans le cas de l'IRM, un contraste est obtenu grâce à l'administration d'agents de contraste contenant des métaux paramagnétiques ou superparamagnétiques qui ont un effet sur la relaxivité des protons de l'eau. Dans le cas de la scintigraphie, le contraste est obtenu par la localisation spécifique d'un composé radiopharmaceutique émettant des rayons gamma ou beta.

Dans le cas du PET, le contraste est obtenu par la localisation spécifique d'un composé radiopharmaceutique émettant des positons.

Dans le cas de l'imagerie CEST on utilise typiquement un métal de déplacement approprié (métal shifteur).

L'invention utilisera pour le ciblage des récepteurs CXCR4 des biovecteurs choisis parmi les bicyclams.

La partie signal comprend au moins un chélate, un grand nombre de chélates pouvant être utilisés.

On pourra utiliser notamment un chélate d'un groupe chélateur linéaire parmi : EDTA, DTPA diéthylènetriaminopentaacétique acide, N-[2-[bis(carboxyméthyl)amino]-3-(4-ethoxyphenyl)propyl]-N-[2-[bis(carboxyméthyl)amino]éthyle]-L-glycine (EOB-DTPA), N,N-bis[2-[bis(carboxyméthyl)amino]éthyle]-L-glutamique acide (DTPA-GLU), N,N-bis[2-[bis(carboxyméthyl)amino]éthyle]-L-lysine (DTPA-LYS), des dérivés mono- ou bis-amide du DTPA, tels que N,N-bis[2-[carboxyméthyl[(méthylcarbamoyl)méthyle]amino]éthyle] glycine (DTPA-BMA), 4-carboxy-5,8,11-tris(carboxyméthyl)-1-phenyl-2-oxa-5,8,11-triazatridecan-13-oic acide (BOPTA),

On pourra utiliser notamment un chélate d'un groupe chélateur macrocyclique parmi 1,4,7,10-tetraazacyclododecan-1,4,7,10-tetraacetic acide (DOTA), 1,4,7,10-tetraazacyclododecan-1,4,7-triacetic acide (DO3A), 10-(2-hydroxypropyl)-1,4,7,10-tetraazacyclododecan-1,4,7-triacetic acide (HPDO3A) 2-methyl-1,4,7,10-tetraazacyclododecan-1,4,7,10-tetraacetic acide (MCTA), ( alpha , alpha alpha ", alpha "')-tetramethyl-1,4,7,10-tetraazacyclododecan- 1,4,7,10-tetraacetic acide (DOTMA), 3,6,9,15-tetraazabicyclo[9.3.1]pentadeca-1(15),11,13-triene-3,6,9-triacetic acide (PCTA), NOTA.

On pourra aussi utiliser des dérivés dans lesquels un ou plusieurs groupes carboxyliques sont sous forme d'un sel, ester, amide correspondant ; ou un composé correspondant dans lequel un ou plusieurs groupes carboxyliques sont remplacés par un groupe phosphonique et/ou phosphinique tel que 4-carboxy-5,11-bis(carboxyméthyl)-1-phényl-12-[(phénylméthoxy)méthyl]-8-(phosphonométhyl)-2-oxa-5,8,11-triazatridecan-13-oic acide, N,N'-[(phosphonométhylimino)di-2,1-ethanediyl]bis[N-(carboxyméthyl)glycine], N,N'-[(phosphonométhylimino)di-2,1-ethanediyl]bis[N-(phosphonométhyl)glycine], N,N'-[(phosphinométhylimino)di-2,1-ethanediyl]bis[N-(carboxyméthyl)glycine], 1,4,7,10-tetraazacyclododecane-1,4,7,10-tetrakis[méthylen(méthylphosphonique)]acide, or 1,4,7,10-tetraazacyclododecane-1,4,7,10-tetrakis[méthyten(méthylphosphinique)]acide

On peut aussi utiliser un chélate d'un groupe chélateur parmi : DOTA gadofluorines, DO3A HPDO3A, TETA, TRITA, HETA, DOTA-NHS, M4DOTA, M4DO3A, PCTA et leurs dérivés 2-benzyl-DOTA,alpha- (2-phenethyl) 1,4,7,10 tetraazacyclododecane-1-acetic-4,7,10-tris (méthylacétique) acide, 2benzyl-cyclohexyldiethylenetriaminepentaacetic acide, 2-benzyl-6methyl-DTPA, 6,6"-bis [N, N, N", N"tétra (carboxyméthyl)aminométhyl)-4'- (3-amino-4-methoxyphenyl)- 2,2' : 6', 2"-terpyridine, N,N'-bis-(pyridoxal-5-phosphate) éthylènediamine- N, N'-diacétique acide (DPDP) et éthylènedinitrilotétrakis (méthylphosphonic) acide (EDTP).

De manière plus large, le ou les chélates formant l'entité signal pourront répondre à la formule du document WO01/60416.

Le couplage de chélates avec des biovecteurs est connue l'art antérieur, et fait généralement intervenir un lien chimique (linker) tel que décrit dans le document WO01/60416. La structure et la nature chimique du linker sont définies pour permettre le couplage chimique entre le biovecteur et le ou les chélates utilisés.

Dans le cas de l'IRM, la relaxivité de ces chélates en imagerie T1 est typiquement de l'ordre de 4 à 20 mMol-1Gd-1s-1. On rappelle que la relaxivité longitudinale r₁ d'un produit de contraste paramagnétique donne la mesure de son efficacité magnétique et permet d'apprécier son influence sur le signal enregistré. En imagerie médicale IRM, les produits de contraste modifient le temps de relaxation des protons, et l'augmentation de la relaxivité obtenue permet d'obtenir un signal plus élevé. Les chélates sont choisis de manière à former des complexes stables avec des ions de métal paramagnétique de nombre atomique 21-29, 42-44, or 58-70, notamment Gd (III), Dy (III), Fe (III), Mn (III) and Mn (II), Tm III pour l'imagerie CEST.

Dans le cas de la scintigraphie, le métal est un radionucléide, notamment ⁹⁹Tc, ¹¹⁷Sn, ¹¹¹In, ⁹⁷Ru, ⁶⁷Ga, ⁶⁸Ga, ⁸⁹Zr, ¹⁷⁷Lu, ⁴⁷Sc, ¹⁰⁵Rh; ¹⁸⁸Re, ⁶⁰Cu, ⁶²Cu, ⁶⁴Cu, ⁶⁷Cu, ⁹⁰Y, ¹⁵⁹Gd, ¹⁴⁹Pr, ¹⁶⁶Ho. Selon une réalisation, le métal est un radionucléide pour l'imagerie PET.

De manière plus large, les biovecteurs de ciblage de CXCR4 peuvent être greffés ou encapsulés dans un système de transport approprié pour une reconnaissance biologique efficace sur le plan diagnostique. De tels systèmes peuvent être des liposomes, micelles, vésicules, microgels, particules lipidiques à multicouches, polymères de saccharides et/ou d'oxyde d'éthylène.

L'invention concerne l'utilisation d'une composition selon les revendications pour le diagnostique d'une pathologie associée à une surexpression ou une sous-expression de récepteurs à des chimiokines.

L'invention concerne aussi l'utilisation des composés décrits précédemment pour le diagnostique de maladies associées à une sur-expression ou une sous-expression de CXCR4 par rapport à un tissu sain, de préférence le diagnostique de tumeurs notamment la détection de métastases tumorales.

L'invention concerne aussi l'utilisation des composés décrits précédemment pour la préparation d'une composition diagnostique destinée au diagnostique de maladies associées à une sur-expression ou une sous-expression de CXCR4 par rapport à un tissu sain, de préférence le diagnostique de tumeurs, notamment des métastases tumorales.

Des exemples d'administration de compositions pour l'imagerie médicale sont décrits dans l'art antérieur, par exemple dans le document WO 0226776. L'agent de diagnostic est administré en quantité suffisante pour une imagerie satisfaisante. En IRM, on utilisera par exemple une dose d'ion métallique de 0,02 à 1,5 mmole/kg de poids corporel.

Des véhicules pharmaceutiquement physiologiquement acceptables permettant de former des compositions diagnostiques (produits de contraste) comprenant les composés décrits précédemment sont connus de l'art antérieur. On utilisera par exemple des sels (sodium, calcium, méglumine), des agents de contrôle du pH (acide acétique, citrique, fumarique, des antioxydants.

Le biovecteur de la présente invention est un bicyclam tels que le composé AMD3100 directement biocouplable ou un de ses dérivés décrits dans : Bioconjugate Chem, 2004, 15,413-423; The American Society for Biochemistry and Molecular Biology, 2003, vol 278, n°47, 47136-47144 ; J.Med.Chem, 1999,42,229-241 ; Chemical Reviews,2003,vol 103,n°9.

On choisira notamment des composés capables de cibler des récepteurs CXCR4 choisis parmi les composés suivants :

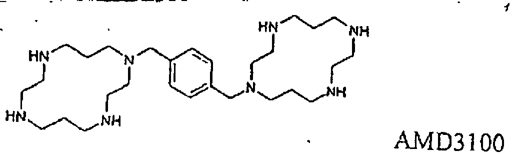

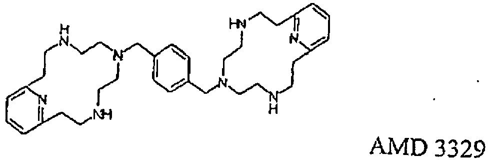

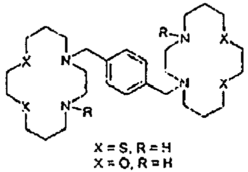

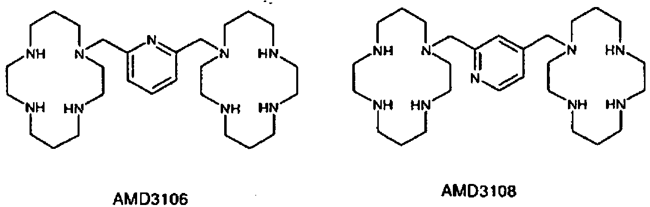

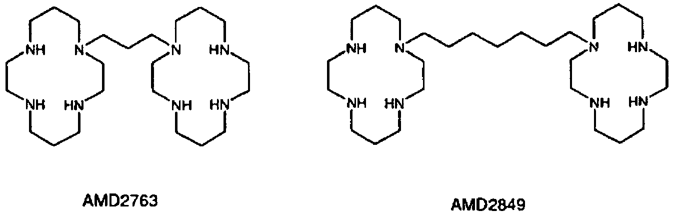

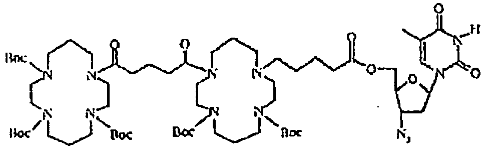

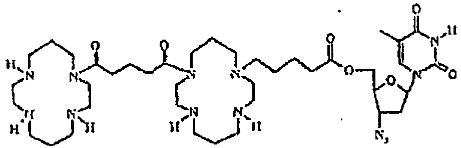

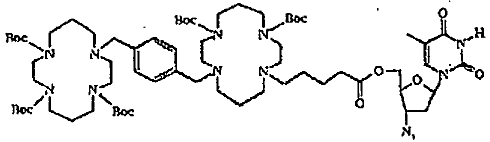

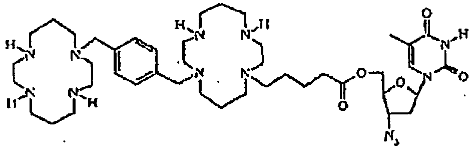

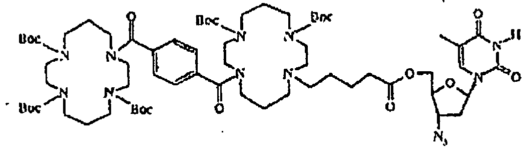

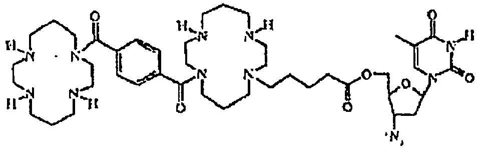

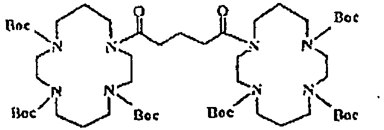

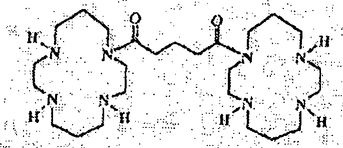

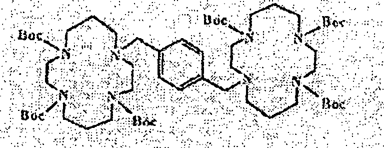

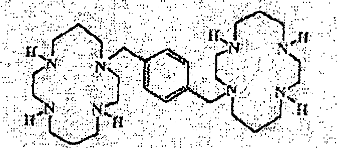

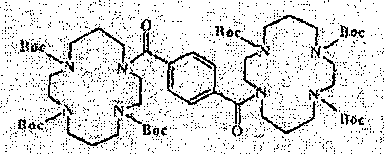

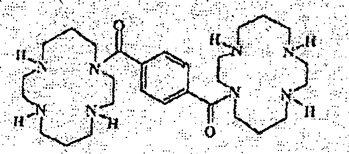

Des composés ciblant CXCR4 sont décrits dans l'art antérieur : le peptide synthétique TN14003 décrit dans Cancer Research, 64, 4302-4308,15 juin 2004, le composé non peptidique KRH-1636 décrit dans PNAS, avril 2003, vol 100, n° 7,4185-4190, des anticorps capables de cibler des récepteurs CXCR4 et des composés décrits dans US 6 667 320, US 2004/0157818 (Yanaka et al), US 2004/0134642 (USA government), US 2004/0102428, US2004/0037825 (Bond et al), US 2004/0019058, US 2003/0220482 (vMIP-II), US 2003/0091569 (Gerritsen et al),US 2002/0039993 (SDF1), US2002/0077339 (Bridger et al), 2004/0009171 (Genentech).

La présente invention comprend un biovecteur bicyclame.

Le couplage entre le biovecteur est réalisé de manière appropriée, typiquement à l'aide d'un lien chimique. Les composés bis-macrocycliques (AMD 3100 et analogues) peuvent être conjugués à des marqueurs paramagnétiques ou radioactifs. Pour cela, il est possible d'utiliser une des fonctions amine secondaire des cycles azotés, les autres fonctions amine étant protégées par un groupement adapté préalablement introduit lors de la synthèse des hétérocycles. Ces groupements sont choisis parmi les groupes protecteurs appropriés des fonctions amines comme les amides, les carbamates. Le composé macrocyclique peut alors être lié au marqueur via la fonction amine secondaire libre à l'aide d'une réaction de couplage peptidique, typiquement par condensation sur un isothiocyanate, par réaction sur un ester de l'acide squarique, par alkylation. Il est également possible d'introduire entre l'azote hétérocyclique et le marqueur un segment de liaison qui permet d'éloigner les deux parties actives de la molécule. Ces segments de liaison sont obtenus par réaction de motifs hétérobifonctionnels de taille et de nature chimique variable comme des chaînes polyméthyléniques, des oligoéthylèneglycols, un aminoacide, un peptide, un noyau aromatique. Ces liens hétérobifonctionnels possèdent une fonction réactive avec la fonction amine secondaire à l'une de leur extrémité comme une fonction acide carboxylique éventuellement activée sous forme d'ester, un halogénure d'alkyle, un ester de l'acide squarique ; et une autre fonction réactive disponible à l'autre extrémité pour recevoir le marqueur. Parmi les fonctions possibles, on peut citer la fonction amine, la fonction acide carboxylique, un thiol, un groupement maléimide, un ester de l'acide squarique. On peut trouver des exemples de segments de liaison hétérobifonctionnels (heterobifonctionnal cross-linkers) dans Bioconjugate Techniques-Greg.T.Hermanson-Academic Press-1996-p228-286. Lorsque la conjugaison des deux entités actives est réalisée, on procède à la déprotection des fonctions bloquées selon les protocoles appropriés de la chimie (voir Protective Groups in Organic Synthesis-second edition-T.W.Green, P.G.M.Wuts-JOHN WILEY SONS-1991 et Protecting Groups-3rd edition-P.J.Kocienski-THIEME-2004).

Le demandeur a étudié notamment les produits suivants.

L'ester terbutylique de l'acide 11-{4-[4,8-Bis-tert-butoxycarbonyl-11-(4-carboxy-butyl)-1,4,8,11tétraaza-cyclotétradec-1-ylméthyl]-benzyl}-1,4,8,11tétraaza-cyclotétradécane-1,4,8-tricarboxylique de formule ( Boc-AMD-(CH₂)₄COOH): 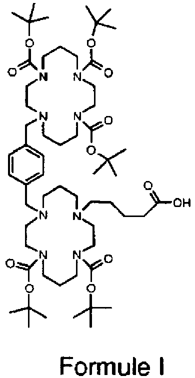 a été préparé selon les données de la littérature (Bioconjugate Chemistry, vol.15, n°2, 2004)

Les exemples suivants sont donnés à titre indicatif,

### Exemple 1 :

### Etape 1 : couplage

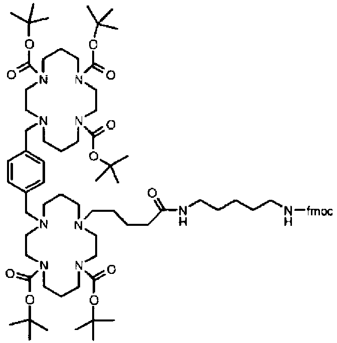

A une solution de 36mg de l'amine mono Fmoc-1,5-Diaminopentane(sous forme de chlorhydrate Novabiochem®), 100mg de l'acide Boc-AMD-(CH₂)₄COOH ( 0,09 mmole) et 32 mg de triéthylamine dans 5 ml de dichlorométhane sont ajoutés 44mg d'agent de couplage BOP (Benzotriazole-1-yl-oxy-tris-(diméthylamino)-phosphoniumhexafluorophosphate). La solution est agitée à température ambiante durant 4h puis lavée successivement avec une solution de NaHCO₃ 5%, KHSO₄ 5% et finalement à l'eau. La phase organique est séchée sur Na₂SO₄ et évaporée à sec. Le produit est purifié sur gel de silice (Elution CH₂Cl₂/MeOH). m /z : ES+ 1600

### Etape 2 : déprotection du Fmoc

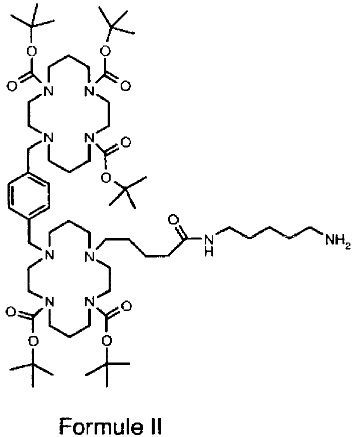

Le composé obtenu à l'étape 1 est dissous dans une solution de pipéridine dans le DMF (20%). La solution est agitée à température ambiante 3H puis évaporée sous vide poussé. L'huile obtenue est lavée à l'éther de pétrole puis chromatographiée sur gel de silice (CH₂Cl₂/MeOH). m /z : ES+ 1189

### Exemple 2 :

### Etape 1 : Ester benzylique de l'acide 5-(1,3-Dioxo-1,3-dihydro-isoindol-2-yl)-2-(1,4,7,10tétraaza-cyclododéc-1-yl)-pentanoique

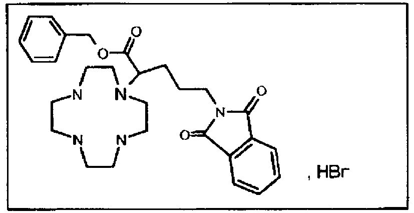

55 g de cyclen base (320 mmol) sont dissous dans 550 mL de CH₃CN, auxquels sont ajoutés goutte à goutte 119.8 g de dérivé bromé (2-Bromo-5-(1,3-dioxo-1,3-dihydro-isoindol-2-yl)-pentanoic acid benzyl ester, 288 mmol) dissous dans 550 mL de CH₃CN. Le milieu est agité à température ambiante 1 nuit. Le précipité est Filtré et lavé abondamment à l'acétonitrile. Obtention de 138 g de produit sous forme d'une poudre. CCM : CH₂Cl₂/ MeOH / NH₄OH à 25% (80/40/3)
Révélation UV et CuSO₄ Rf : 0,3

### Etape 2 : Ester benzyligue de l'acide 5-(1,3-Dioxo-1,3-dihydro-isoindol-2-yl)-2-(4,7,10-tris-éthoxycarbonylméthyl-1,4,7,10tétraaza-cyclododéc-1-yl)-pentanoique

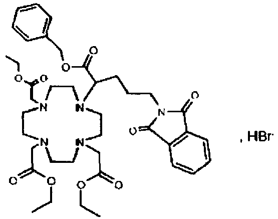

A une solution de 59,1 g d'ethylbromoacétate (Aldrich®, 358 mmol) dans le CH₃CN (1,1 l), sont additionnées 60 g du composé obtenu à l'étape 1 (102 mmol) et 50,1 g de Na₂CO₃ (464 mmol). Le milieu réactionnel est chauffé à 80°C sous couverture d'argon pendant une nuit. Après élimination du précipité, le filtrat est concentré et lavé abondamment au CH₃CN. Le produit est cristallisé dans CH₃CN par ajout goutte à goutte d'Et₂O. Obtention de 89,8 g de produit sous forme d'un solide blanc. CCM : CH₂Cl₂ / MeOH (9/1). Révélation UV et KMnO₄.
Rf : 0,4

### Etape 3 : Acide 5-Amino-2-(4,7,10-tris-carboxyméthyl-1,4,7,10tétraaza-cyclododéc-1-yl)-pentanoique

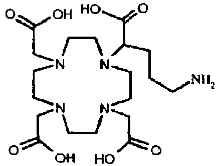

Dans le réacteur de 5 litres, on chauffe au reflux une solution de 54 g de composé obtenu à l'étape 2 (64 mmol) dans l'acide chlorhydrique 37 % (1,8 l), pendant une nuit. Après refroidissement et filtration, le filtrat est concentré et purifié sur silice silanisée (élution à l'eau). Après évaporation sous pression réduite, le produit est lavé à l'éther. Obtention de 45 g de produit sous forme d'un solide blanc. Le produit est désalifié par passage sur résine OH⁻. On isole 30 g de produit sous forme de cristaux blancs.
HPLC : Hypercarb® 5µ, 200X4,6, 250Å
Solvant A : acide sulfurique 0,037 N ; Solvant B : CH₃CN ; Détection UV à 201 nm ;
Tr: 18 min.

### Etape 4: Complexe de gadolinium de l'Acide 5-Amino-2-(4,7,10-tris-carboxyméthyl-1,4,7,10tétraaza-cyclododéc-1-yl) -pentanoique

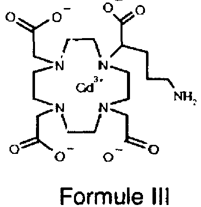

7,2 g du composé obtenu à l'étape 3 (16 mmol) sont dissous dans 70 mL d'eau et le pH est ajusté à 5,5 par ajout d'acide chlorhydrique 6 N. On ajoute 2,9 g de Gd₂O₃ (8 mmol), et chauffe à 80°C le milieu réactionnel. Le pH de la solution augmente régulièrement, et doit être maintenu entre 5,2 et 5,7 par ajout goutte à goutte d'acide chlorhydrique 6 N. Après deux heures, le pH se stabilise à 5,7. Le léger trouble est filtré sur filtre Whatman® et le filtrat est concentré. Obtention de 11,1 g de produit sous forme de paillettes blanches. HPLC : Hypercarb® 5µ, 200X4,6, 250Å.Solvant A : acide sulfurique 0,037 N.Solvant B : CH₃CN.Détection UV à 201 nm. Tr : 10 min.

### Etape 5 : Complexe de gadolinium de l'Acide 5-(2-Ethoxy-3,4-dioxo-cyclobut-1-énylamino)-2-(4,7,10-tris-carboxyméthyl-1,4,7,10tétraaza-cyclododéc-1-yl)-pentanoique

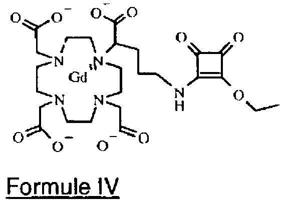

8 g de composé obtenu à l'étape 4 sont séchés par distillation azéotropique au toluène, puis mis en suspension dans 90 ml de DMSO anhydre sous couverture d'argon. 2,8 ml de Et₃N séchée sur tamis (1,7 éq) et 5 g de diéthyisquarate (Aldrich®, 2,5 éq.) sont ensuite ajoutés. Le milieu est agité à température ambiante sous couverture d'argon pendant 1 heure. Le mélange est précipité dans 600 ml d'éther. Le solide obtenu est filtré, puis lavé au dichlorométhane. Après filtration et séchage, 7,5 g d'un solide blanc (rendement de 81,5 %) sont récupérés. HPLC : Symmetry C18, 5 , 250X4,6, 100Å. A : eau TFA, pH = 2,7. B : CH₃CN. Détection à 201 et 254 nm. Tr : 19,8 min.

### Exemple 3 :

### Etape 1 : couplage

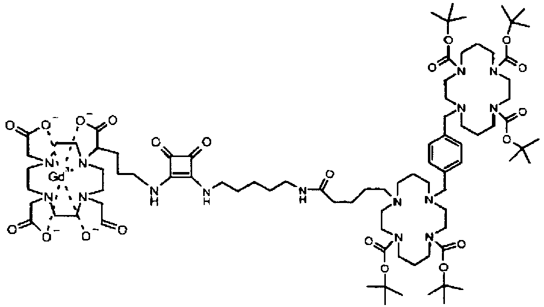

Le composé obtenu à l'étape 5 de l'exemple 2 (100mg, 1,35 10⁻⁴ mole) est dissous dans 10ml de solution aqueuse de Na₂CO₃ pH 9,4. Le composé obtenu à l'étape 2 de l'exemple 1 (176 mg) est introduit en maintenant le pH à 9,4 par ajout de Na₂CO₃. Quelques gouttes de DMF sont ajoutées jusqu'à dissolution complète. Après 48h de réaction à température ambiante, le milieu est précipité dans un mélange éthanol/éther éthylique. Le précipité est filtré puis Séché. m /z : ES+ 1882

### Etape 2 : déprotection des terbutyles

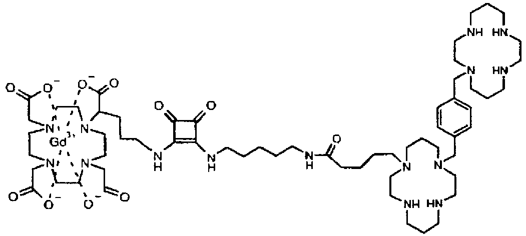

Le composé obtenu à l'étape 1 est dissous dans un mélange de 10 cm³ de TFA/TIS/H₂O dans les proportions 90/5/5. Le milieu est agité 5h à température ambiante puis le solvant est évaporé sous pression réduite. Le résidu est repris dans l'éther éthylique et le précipité est filtré puis séché. Le produit est ensuite purifié par HPLC préparative sur une colonne Symmetry® avec un éluant constitué d'eau/TFA PH 3/ CH₃CN. m /z : ES+ 1381

### Exemple 4 :

### Etape 1 : couplage

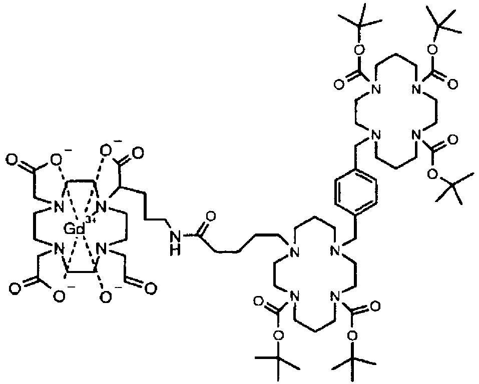

A 0,1 g du complexe préparé à l'étape 4 de l'exemple 2, et 197mg (soit 1,1 éq) de Boc-AMD-(CH₂)₄COOH sont dissous dans 25 mL d'eau/DMF. 405mg d'EDCI (soit 1,3 éq) et 0,01g de HOBt sont ajoutés. Le milieu réactionnel est chauffé à 40°C pendant 12 heures et le pH est maintenu à environ 6 par ajout de quelques gouttes de NaOH 2N. Purification : Le milieu réactionnel est précipité dans 250 mL d'acétone. Le produit est filtré et le solide blanc obtenu est séché sur P₂O₅.
SM : ES+, M/Z= 1702 avec z=1

### Etape 2 : déprotection

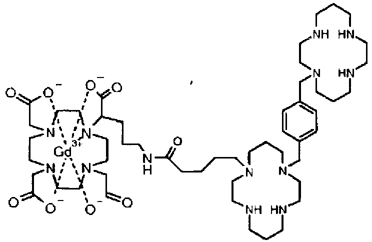

Selon le mode opératoire de l'étape 2 de l'exemple 3 au départ du composé obtenu à l'étape 1. SM : ES+, M/Z= 1202 avec z=1

### Exemple 5 :

### Etape 1 : Ester méthylique de l'acide (13-Bromo-6,9-bis-éthoxycarbonylméthyl-3,6,9,15-tétraaza-bicyclo[9.3.1]pentadéca-1(14),11(15),12-trièn-3-yl)-acétique

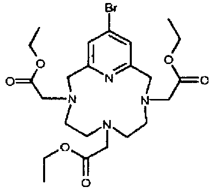

22 g de 13-bromo-3,6,9,15-tétraazabicyclo[9.3.1.]pentadéca-1(15),11,13-triène obtenus selonJ. Heterocyclic Chem. 27, 1990, pages 167-169, sont introduits dans 440 ml de CH₃CN en présence de 48 g de K₂CO₃ calciné et le mélange est maintenu à 80° C pendant 1 h avant l'addition d'une solution de 50 g de Bromoacétate d'éthyle dans 100 ml de CH₃CN ; le milieu réactionnel est alors agité 20 h à 80° C puis refroidi à température ambiante, filtré et le solvant est évaporé. Le résidu est repris par 500 ml d'une solution aqueuse de HCl 1N en présence d'un volume d'éther diéthylique. Après séparation de la phase organique, la phase aqueuse est neutralisée par NaHCO₃ puis extraite par CH₂Cl₂. Après lavage à l'eau puis séchage sur sulfate de magnésium, la phase organique est concentrée et le résidu est purifié sur colonne de silice (Merck^{®} 500 g, d = 10 cm) en éluant par CH₃COOC₂H₅. m = 20.9 g ; m /z : ES+ 544,6

### Etape 2: Ester méthylique de l'acide [13-(3-tert-Butoxycarbonylamino-propènyl)-6,9-bis-éthoxycarbonylméthyl-3,6,9,15-tétraaza-bicyclo[9.3.1]pentadéca-1(14),11(15),12-trièn-3-yl]-acétique

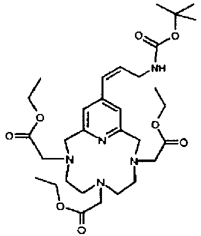

On ajoute 6 g de 3-(tert-butyloxycarbonylamino)-propène, 10 ml de triéthylamine, puis 800mg de triphénylphosphine et enfin 400 mg d'acétate de palladium à une solution de 5g du composé obtenu à l'étape a) dissous dans 100 ml de toluène. Après chauffage à 80° C pendant une nuit sous atmosphère inerte, le milieu est évaporé et le résidu est repris par une solution aqueuse d'acide chlorhydrique (pH = 1). La phase aqueuse est lavée avec 1 volume d'éther diéthylique puis de toluène avant d'être amenée à pH 6 par addition de NaOH (1 N).

Après extraction de la solution aqueuse par CH₂Cl₂, la phase organique séchée sur sulfate de magnésium est évaporée. On obtient une huile marron qui est chromatographiée sur gel de silice. m = 2,8 g. m /z : ES+ 621

### Etape 3: Ester éthylique de l'acide [13-(3-tert-Butoxycarbonylamino-propyl)-6,9-bis-éthoxycarbonylméthyl-3,6,9,15-tétraaza-bicyclo[9.3.1]pentadéca-1(14),11(15),12-trièn-3-yl]-acétique

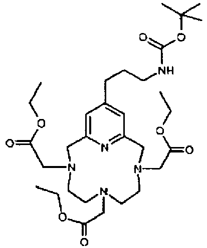

A 2 g du composé obtenu à l'étape b) dissous dans 80 ml de CH₃OH, on ajoute 200mg de catalyseur charbon palladié à 10% puis le milieu réactionnel est agité durant 2 h 30 à 20° C sous 4.10⁵ Pa d'hydrogène. Après filtration sur Clarcel^{®}, le solvant est évaporé et on obtient 1,8 g d'huile après chromatographie sur gel de silice. m /z : ES+ 623

### Etape 4: Acide [13-(3-tert-Butoxycarbonylamino-propyl)-6,9-bis-carboxyméthyl-3,6,9,15-tétraaza-bicyclo[9.3.1]pentadéca-1(14),11(15),12-trien-3-yl]-acétique

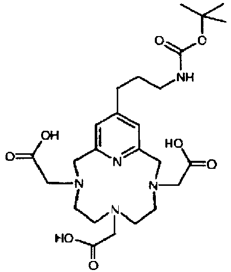

1g du composé obtenu à l'étape c) dissous dans 20 ml d'une solution aqueuse de NaOH 5N et 20 ml de CH₃OH sont chauffés à 70° C pendans 18 h. Après concentration du milieu réactionnel, le résidu est repris dans l'eau et la solution, amenée à pH 5,5-6 par quelques gouttes d'acide acétique, est concentrée avant d'être purifiée par chromatographie sur une colonne (d = 15 cm) contenant 50 g de silice silanisée (Merck^{®} 0,063 - 0,200 µm) en éluant à l'eau. Après concentration à sec on obtient 480mg de cristaux blancs. m /z : ES- 536,5

### Etape 5: Complexe de gadolinium de l'acide [13-(3-tert-Butoxycarbonylamino-propyl)-6,9-bis-carboxyméthyl-3,6,9,15-tétraaza-bicyclo[9.3.1]pentadéca-1 14),11(15),12-trièn-3-yl]-acétique

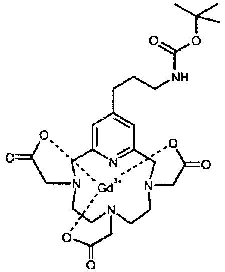

300mg du composé obtenu à l'étape d) sont dissous dans 10 ml d'eau puis on ajoute en une fois 100mg de Gd₂O₃ et l'ensemble est chauffé à 60° C durant 3 h 45 min en maintenant le pH entre 5,5 et 6 par addition d'une solution aqueuse de NaOH 1 N. Après filtration, le milieu réactionnel est évaporé et le résidu est cristallisé dans l'éthanol. Après traitement par une résine Chelex^{®} 100 (Bio-Rad) on obtient 320mg de cristaux blancs. m /z : ES- 691

### Etape 6: Complexe de gadolinium de l'acide [13-(3-Amino-propyl)-6,9-bis-carboxyméthyl-3,6,9,15-tétraaza-bicyclo[9.3.1]pentadéca-1(14),11(15),12-trièn-3-yl]-acétique

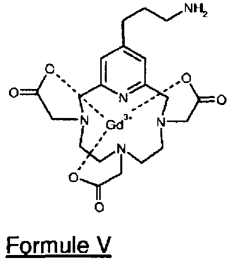

On maintient 3 h sous agitation une solution de 300mg du complexe obtenu à l'étape e) dans 18ml de CF₃COOH à 25° C avant d'éliminer le liquide sous pression réduite. Le résidu est repris dans l'éther diéthylique et la suspension filtrée. Après élimination du solvant, le résidu est introduit par portions dans une suspension d'au moins 1 ml de résine anionique faible (OH⁻) dans 5 ml d'eau ; en fin d'addition le pH, stable, doit être de 8 à 8,5. La résine est alors séparée par filtration, le solvant éliminé et le résidu précipité par addition d'éther éthylique.m= 200mg. m /z : ES+ 593

### Exemple 6 :

### Etape 1 : couplage

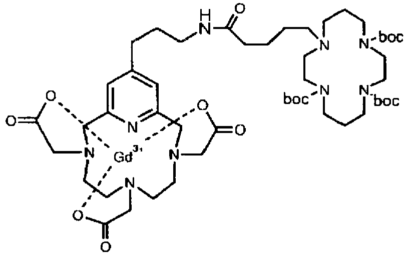

Selon le mode opératoire de l'étape 1 de l'exemple 4 au départ de 100mg du complexe de gadolinium préparé à l'étape 6 de l'exemple 5 et 102 mg de l'ester terbutilyque de l'acide 11-(4-Carboxy-butyl)-1,4,8,11tétraaza-cyclotétradécane-1,4,8-tricarboxylique préparé selon les données de la littérature (J.of Medicinal chemistry, 1999, vol 42, n °2 p229-241). m/z:ES+1176

### Etape 2 : déprotection

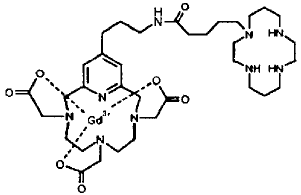

Selon le mode opératoire de l'étape 2 de l'exemple 4 au départ du composé obtenu à l'étape 1. m/z : ES+ 875

### Exemple 7 :

### Etape 1 : couplage

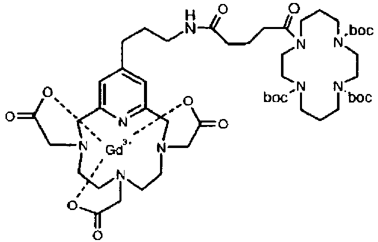

Selon le mode opératoire de l'étape 1 de l'exemple 4 au départ de 100mg du complexe de gadolinium préparé à l'étape 6 de l'exemple 5 et 104 mg de l'ester terbutilyque de l'acide 11-(4-Carboxy-butyryl)-1,4,8,11 tétraaza-cyclotétradécane-1,4,8- tricarboxylique préparé selon les données de la littérature (J.of Medicinal chemistry, 1999, vol 42, n°2 p229-241). m/z:ES+1190

### Etape 2 : déprotection

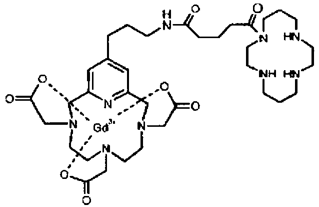

Selon le mode opératoire de l'étape 2 de l'exemple 4 au départ du composé obtenu à l'étape 1.
m /z : ES+ 889

### Exemple 8 :

Couplage du composé obtenu à l'étape 2 de l'exemple 1 avec les complexes bimétalliques décrits dans le brevet WO2004/112839.
- exemple n°3 pages 93-94 et 91 à 93 de formule VI : 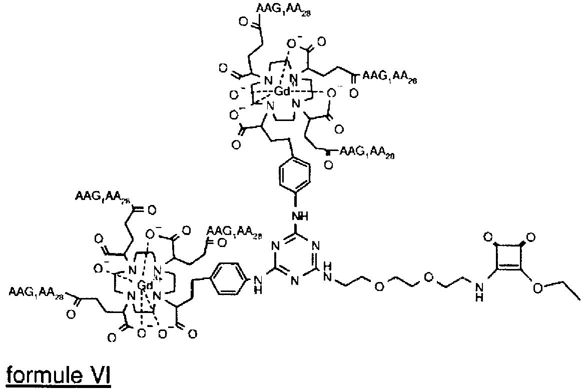
- exemple 6 p 98 à 101, et 96 à 98 de formule VII :

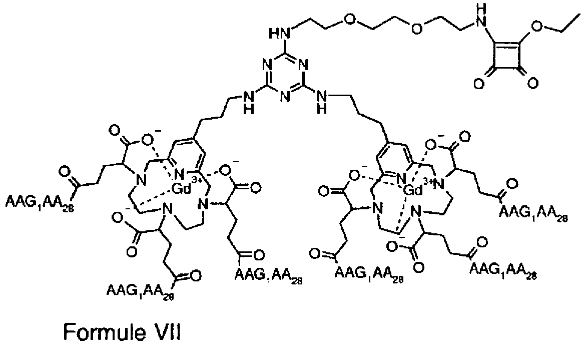

Avec AAG1AA28=

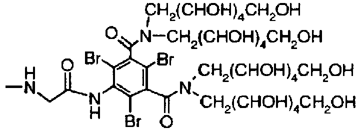

### Etape 1 : Couplage

| Composé | Poids Mol. | Qté engagée | Nbre de Mol. |
|---|---|---|---|
| Formule VI | 8802,59 | 300mg | 3,41 10⁻⁵ |
| Formule II | 1187,67 | 47,5mg | 410⁻⁵ |
| Formule VII | 8632,43 | 300mg | 3,48 10⁻⁵ |
| Formule II | 1187,67 | 50mg | 4,18 10⁻⁵ |

300mg d'un composé bimétallique précédemment décrit (formule VI ou VII), sont dissous dans 2 ml d'eau. Le pH est amené à 9,5 par addition de Na₂CO₃. 1,2 équivalent du composé décrit étape 2 de l'exemple 1 (formule II) sont ajoutés. Le milieu réactionnel est agité à température ambiante pendant 3 jours puis est précipité dans l'éthanol.

### Etape 2 : déprotection

Le composé obtenu selon l'étape 1 est dissous dans 10ml du mélange acide trifluoroacétique/eau/triisopropylsilane (90/5/5). Après 4h à température ambiante sous agitation, le TFA est éliminé par évaporation sous vide. Le milieu réactionnel est précipité dans l'éther. Le produit obtenu par filtration est ensuite purifié par HPLC préparative. HPLC : Colonne Superpher Select B ® ;eau-TFA pH 3 / CH₃CN

| Composé | Poids Mol. | Qté obtenue | M/Z ES +. |
|---|---|---|---|
| VIII | 9443,61 | 110mg | 9445 |
| IX | 9273,44 | 90mg | 9276 |

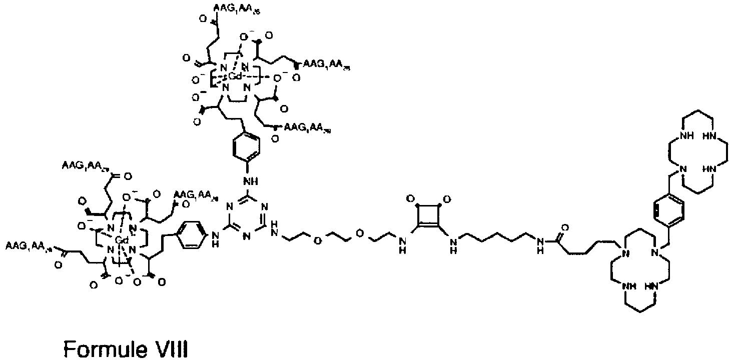

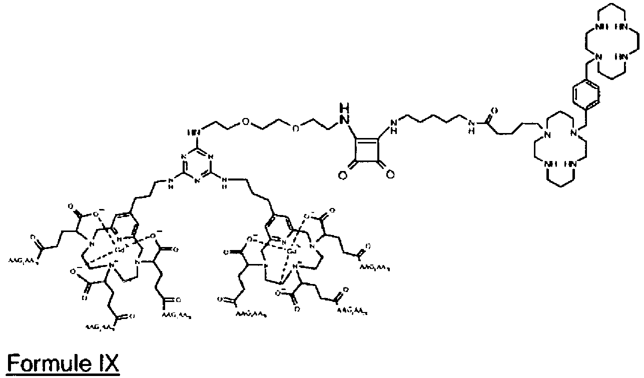

### Exemple 9: Comparatif

### Synthèse de cyclopeptides de formules X,

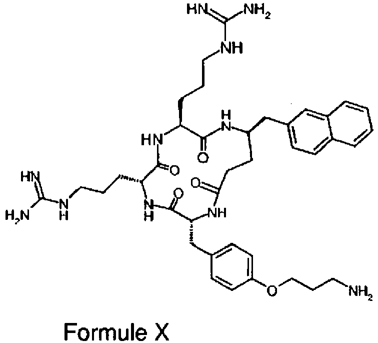

Le peptide a été préparé selon la méthode décrite dans J.Of Medicinal chemistry, 2005, vol.48, N°9, p3280-3289. La synthèse sur phase solide a été réalisée sur résine Chlorotrityle en utilisant des aminoacides protégés par le groupement Fmoc.

La tyrosine utilisée dans la publication originale a été remplacée par un analogue substitué de formule :

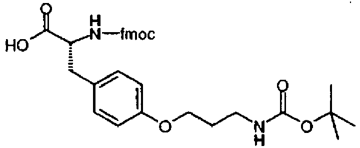

Ce composé a été préparé selon la méthode décrite dans Tetrahedron Letters vol.36, N°35, p6193-6196, 1995.

### Exemple 10: Comparatif

Couplage du cyclopeptide de formule X sur des nanoparticules d'oxyde de Fe .

Les nanoparticules ont été préparées selon les méthodes décrites dans le brevet WO2004/058 275 (US 2004/253181) exemples 8 et 9 pour la préparation des solutions colloïdales de particules magnétiques et exemples 10 à 12 pour la complexation des particules magnétiques par un revêtement gembisphonate de l'exemple 1 de WO2004/058 275.

Le couplage se fait de manière analogue à celui décrit pour les exemples 13 à 15 du WO2004/058 275. A 50 cm³ de solution de nanoparticule d'oxyde de Fer sont ajoutés progressivement en maintenant le PH vers 7 une solution de 100mg de peptide de formule X et 110mg d'EDCI (1-(3-diméthylaminopropyl)-3-éthylcarbodiimide chlorhydrate). Le pH de la solution est ramené à 6,5 par NaOH (0,1N). La solution est Agitée puis. Filtrée sur une membrane de porosité 0,22µm (STERICUP Millipore®).La solution est ensuite Ultrafiltrée sur membrane de 30KD. [Fe] = 0,250 M/L
Taille PCS = 28nm
Taux de greffage [ composé A / Fe ] = 1,65 % mole/mole
Taux de greffage [Peptide/ composé A ] = 31 %

### Exemple 11: Comparatif

### Couplage de l'amine R-NH2, N-N'-[bis(2,3,4,5,6-pentahydroxyhexyl)] 2,4,6-tribromo 5-(glycylamino)isophtlamide formule :

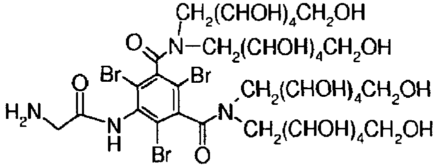

peut être préparée selon le mode opératoire décrit dans le Brevet : EP 0 922 700 A1.

A 50 cm³ de solution obtenue exemple 10 à température ambiante est ajouté une solution constituée de 2,5 g de l'amine R-NH2 dans 10 cm3 d'eau. Le PH est ajusté à 7 par ajout de NaOH 0,1M. 1 g d'EDCI est ajouté et la solution est agitée 3h à température ambiante. Le PH est ajusté à 7 et le mélange est agité 1 nuit à température ambiante. Après filtration sur une membrane de porosité 0,22µm, la solution est ultrafiltrée sur une membrane de seuil de coupure 30KD.
[Fe] = 0,228 M/L Taille PCS = 28,3nm
Taux de greffage [ composé A / Fe ] = 1,62% mole/mole
Taux de greffage [Peptide/ composé A ] = 32 %
Taux de greffage [R-NH2/ composé A ] = 62 %

### Exemple 12: Comparatif

### Couplage de l'aminoPEG 750 ( O-(2-aminoéthyl)-O'-méthylpolyéthylèneglycol 750 FLUKA®)

A 50 cm³ de solution obtenue exemple 10 à température ambiante est ajouté une solution constituée de 2 g de l'aminoPEG 750 dans 10 cm3 d'eau. Le PH est ajusté à 7 par ajout de NaOH 0,1M. 1 g d'EDCI est ajouté et la solution est agitée 3h à température ambiante. Le PH est ajusté à 7 et le mélange est agité 1 nuit à température ambiante. Après filtration sur une membrane de porosité 0,22µm, la solution est ultrafiltrée sur une membrane de seuil de coupure 30KD.
[Fe] = 0,212 M/L Taille PCS = 27,8nm
Taux de greffage [ composé A / Fe ] = 1,70% mole/mole
Taux de greffage [Peptide/ composé A ] = 30 %
Taux de greffage [R-NH2/ composé A ] = 62 %

Le demandeur a mis au point les protocoles biologiques suivants pour mesurer l'efficacité des produits.

Affinité / spécificité *in vitro* pour CXCR-4 : TEST biologique N°1 : test de fixation basé sur une reconnaissance, par l'entité signal vectorisée, d'une cellule exprimant la protéine cible (HL-60). La spécificité de reconnaissance est évaluée grâce à des expériences de compétition montrant un rôle inhibiteur direct d'un excès de vecteur libre sur la fixation du prototype testé. La spécificité est également être validée par comparaison avec des dosages obtenus avec la molécule de référence non vectorisée. La cible cellulaire choisie est la cellule pro-myéloïde humaine HL-60 décrite dans la littérature comme fortement positive pour CXCR-4 [R. Möhle et al. : « The chimiokine receptor CXCR-4 is expressed on CD34+ hematopoietic progenitor and leukemic cells and mediates transendothelial migration induced by stromal cell-derived factor-1 » Blood 12 : 4523-4530 (1998)].

### Modèle de fixation sur cellules HL-60 :

- Cellules :: HL-60
- Délais :: Temps d'incubation en présence du produit test : cinétique de 0,5h à 4 h à 37°C.
- Contrôles :: Vérification du taux d'expression en CXCR-4 des HL-60 par cytométrie en flux.
Incubation des cellules en présence de la molécule de référence non vectorisée.
- Spécificité :: Inhibition de la fixation des prototypes par un excès de vecteur libre ou comparaison avec les résultats obtenus avec la molécule de référence non vectorisée.
- Résultats :: Quantités de Fe ou de Gd présentes dans les culots cellulaires après dosage par ICP-AES ou ICP-MS respectivement (quantités rapportées en million de cellules ou en mg de protéines).

L'analyse des résultats prend en compte la quantité de Fe ou de Gd trouvée avec les particules vectorisées rapportée à celle détectée :
1/ en présence du contrastophore non vectorisé
2/ en présence du prototype vectorisé et d'un excès de ligand (x100). Ces résultats permettent de déterminer l'apport du vecteur sur le mécanisme de reconnaissance et la spécificité de reconnaissance est étudiée par les expériences de compétition avec excès de vecteur libre.

Affinité / spécificité *in vitro* pour CXCR-4 : TEST biologique N°2 : test de fixation basé sur une reconnaissance, par l'entité signal vectorisée, d'une cellule exprimant la protéine cible avec, comme contrôle, la même cellule mais négative pour le récepteur. La spécificité de reconnaissance est évaluée grâce à des expériences comparant les résultats obtenus sur cellules positives à ceux obtenus sur cellules négatives. La cible cellulaire choisie est la cellule CHO (Chinese Hamster Ovary) négative pour l'expression de CXCR-4.

### Modèle de fixation / internalisation sur cellules CHO :

| | |
|---|---|
| Cellules : | CHO positives et négatives pour le récepteur humain CXCR-4 |
| Délais : | Temps d'incubation en présence du produit test : cinétique de 0,5h à 2 h à 37°C. |
| Contrôles : | Incubation des cellules en présence de la molécule de référence non vectorisée. |
| Spécificité : | Comparaison des différents groupes (cellules CHO positives et négatives/produit vectorisé et produit de référence) |

## Revendications

1. Composé comprenant d'une part une partie de ciblage de récepteurs CXCR4, et d'autre part une partie de détection capable d'être identifiée par une méthode d'imagerie médicale, dans lequel la partie de détection comprend un chélate linéaire ou macrocyclique d'ion de métal paramagnétique ou de radionucléide ou de métal shifteur et la partie ciblage est un bicyclam.

2. Composé selon la revendication 1 dans lequel la partie de détection est un chélate linéaire ou macrocyclique d'ion de métal paramagnétique ou de métal shifteur.

3. Composé selon la revendication 1 ou 2 dans lequel le groupe chélateur est choisi parmi les composés DTPA, DTPA BMA, DTPA BOPTA, DO3A, TETA, TRITA, HETA, DOTA, TETA, NOTA, PCTA et leurs dérivés, de préférence le PCTA.

4. Composition diagnostique comprenant un composé selon l'une des revendications précédentes et un véhicule pharmaceutiquement acceptable.

5. Utilisation d'un composé selon les revendications 1 à 3 pour la fabrication d'une composition destinée au diagnostic d'une pathologie associée à une surexpression ou une sous-expression de récepteurs CXCR4, de préférence le diagnostic de tumeurs, notamment des métastases tumorales.

6. Utilisation selon la revendication 5, caractérisée en que la composition comprend un véhicule pharmaceutiquement acceptable.

## Patentansprüche

1. Verbindung, die einerseits einen CXCR4-Rezeptor-Adressierungsabschnitt und andererseits einen Nachweisabschnitt, der mittels eines medizinischen Bildgebungsverfahrens identifiziert werden kann, umfasst, wobei der Nachweisabschnitt ein geradkettiges oder makrocyclisches Chelat eines Ions eines paramagnetischen Metalls oder Radionukleids oder Shifter-Metalls umfasst und der Adressierungsabschnitt ein Bicyclam ist.

2. Verbindung nach Anspruch 1, worin der Nachweisabschnitt ein geradkettiges oder makrocyclisches Chelat eines Ions eines paramagnetischen Metalls oder eines Shifter-Metalls handelt ist.

3. Verbindung nach Anspruch 1 oder 2, wobei die Chelatisierungsgruppe aus der Reihe der Verbindungen DTPA, DTPA BMA, DTPA BOPTA, DO3A, TETA, TRITA, HETA, DOTA, TETA, NOTA, PCTA und ihren Derivaten, vorzugweise PCTA, ausgewählt ist.

4. Diagnostische Zusammensetzung, die eine Verbindung nach einem der vorhergehenden Ansprüche und einen pharmazeutisch unbedenklichen Grundstoff umfasst.

5. Verwendung einer Verbindung nach den Ansprüchen 1 bis 3 zur Herstellung einer Zusammensetzung für die Diagnose einer mit Überexpression oder Unterexpression von CXCR4-Rezeptoren assoziierten Pathologie, vorzugweise für die Diagnose von Tumoren, insbesondere Tumormetastasen.

6. Verwendung nach Anspruch 5, **dadurch gekennzeichnet, dass** die Zusammensetzung einen pharmazeutisch unbedenklichen Grundstoff umfasst.

## Claims

1. Compound comprising, firstly, a CXCR4-receptor-targeting component and, secondly, a detection component capable of being identified by a medical imaging method, in which the detection component comprises a linear or macrocyclic chelate of an ion of a paramagnetic metal or of a radionuclide or a shift metal and the targeting component is a bicyclam.

2. Compound according to Claim 1, in which the detection component is a linear or macrocyclic chelate of an ion of a paramagnetic metal or of a shift metal.

3. Compound according to Claim 1 or 2, in which the chelating group is chosen from the compounds DTPA, DTPA BMA, DTPA BOPTA, DO3A, TETA, TRITA, HETA, DOTA, TETA, NOTA, PCTA and derivatives thereof, preferably PCTA.

4. Diagnostic composition comprising a compound according to one of the preceding claims and a pharmaceutically acceptable carrier.

5. Use of a compound according to Claims 1 to 3, for the manufacture of a composition intended for the diagnosis of a pathology associated with an overexpression or an underexpression of CXCR4 receptors, preferably the diagnosis of tumours, in particular tumour metastases.

6. Use according to Claim 5, **characterized in that** the composition comprises a pharmaceutically acceptable carrier.
